# EUROPEAN PATENT SPECIFICATION

(11) **EP 3 222 167 B1**
(45) Date of publication and mention of the grant of the patent: **23.01.2019**
(21) Application number: 17000481.6
(22) Date of filing: 23.03.2017
(51) Int. Cl.: A45F 3/04, A45C 13/24, A45C 13/38, A61F 17/00

(54) **PROFESSIONAL BACKPACK, PARTICULARLY FOR TECHNICAL AND/OR SPECIALIST MEDICAL RESCUE MISSIONS**
PROFESSIONELLER RUCKSACK, INSBESONDERE FÜR RETTUNGSMISSIONEN SEITENS TECHNISCHER UND/ODER MEDIZINISCHER SPEZIALISTEN
SAC À DOS PROFESSIONNEL, EN PARTICULIER POUR DES MISSIONS DE SAUVETAGE MÉDICAL TECHNIQUES ET/OU SPÉCIALISTES

(30) Priority: 25.03.2016 IT UA20162027
(43) Date of publication of application: 27.09.2017
(73) Proprietor: Ferrino & C. S.p.A., 10099 San Mauro Torinese (Torino) (IT)
(72) Inventor: Aimo-Boot, Marco, 10072 Caselle Torinese (Torino) (IT)
(74) Representative: Cian, Paolo

(56) References cited:
- US-A1- 2008 291 004
- US-A1- 2011 315 284
- US-A1- 2013 106 607
- US-A1- 2014 326 771
- US-A1- 2016 030 261

## Description

### Field of the Invention

The present invention relates to a professional backpack, in particular for hazardous missions aimed at saving human lives and/or for technical assistance on land, in speleo-alpine-riverine environment as well as in helicopter rescue activities.

### State of the art

Backpacks used in hazardous missions often contain life-saving medicines and essential first aid medical products for use by qualified medical and paramedical staff. However, these need to be replaced cyclically, to be stored at the correct temperatures until their use, or restored after their use in the previous mission.

In the circumstances in which a service shift begins, it is very important to carry out the accurate check (checklist) of the equipment in order to avoid making a mission ineffective.

Additionally, missions may be performed in particularly unfavorable environments.

US-2013/106607-A1 discloses an intelligent portable carrier device, for example a portable container, comprising a housing provided with a closable opening, for transporting items such as sterile implants and biologic products or other biological materials. This carrier device uses product identification technology, such as radio-frequency identification (RFID) tags and readers, to uniquely identify the regulated products as they are added to or removed from the intelligent portable carrier device. The carrier device may also be configured to monitor and report temperature and other environmental conditions associated with it.

### Summary of the Invention

The object of the present invention is to provide a professional backpack, particularly for hazardous missions, which is adapted to provide adequate support to human life-saving staff and, in any case, to hazardous missions in general.

The idea on which the present invention is based is to provide a backpack intended to perform a series of features in connection with its movement.

In addition, it is provided with removable portions for containing material in connection with the missions in which the backpack itself is involved, and one of said features involves detecting the presence of such removable portions.

An object of the present invention is a professional backpack, in particular for hazardous missions, according to claim 1.

The dependent claims describe preferred embodiments of the invention, and form an integral part of the present description.

### Brief description of the Figures

Further characteristics and advantages of the invention will become more apparent in the light of the following detailed description of preferred, but not exclusive, embodiments of a professional backpack, in particular for hazardous missions, shown by way of non-limiting example, with the aid of the appended drawings in which:

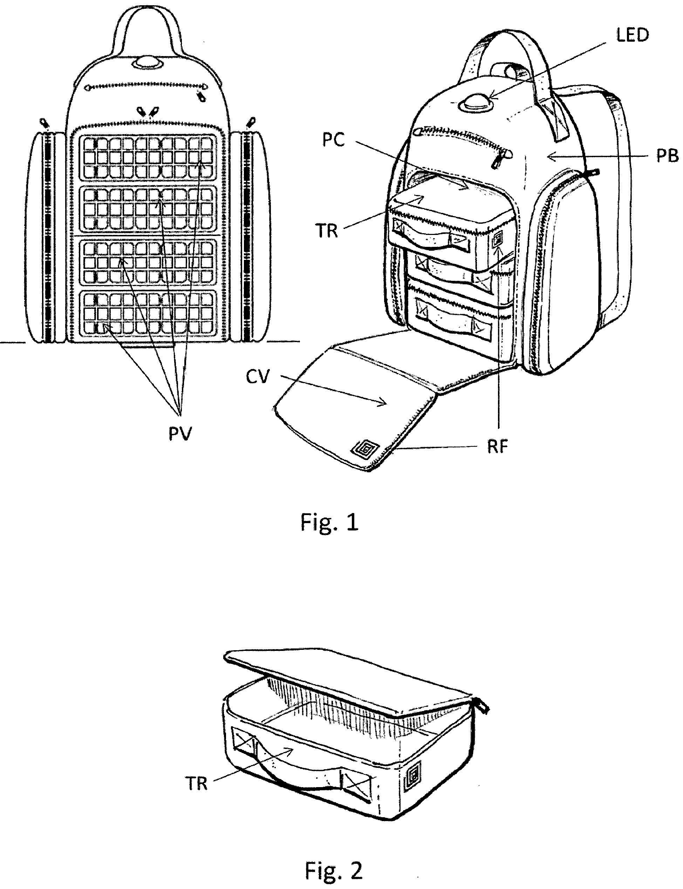

Figure 1 is a perspective view of a backpack according to the present invention in a configuration in which a main backpack compartment is open,
Figure 2 shows a portion of the backpack removed from the main compartment of Figure 1,
Figure 3 shows a side view of the backpack explaining opening of the main compartment of Figure 1,
Figure 4 shows a front view of the backpack of Figure 1 in which the main compartment is in its fully closed condition,
Figure 5 shows a block diagram of a processing unit associated with the backpack of the previous figures, and with portable devices,
Figure 6 shows a perspective view of a transport trolley that can be associated with the backpack of the previous figures.

The same reference numerals and letters in the figures identify the same elements or components.

### Detailed description of a preferred embodiment of the invention

The main structure of the backpack PB of the present invention defines a large main compartment PC that can be accessed from a front opening that can be resealed preferably by means of one or two zippers symmetrical to each other with respect to the longitudinal axis of the backpack. The zipper, its sliders, and the corresponding flexible pulls are preferably of fluorescent yellow to allow them to be quickly detected at the time of opening and closing.

Opening of the zipper/s allows a soft or semi-rigid wall of the backpack to be folded downwards in order to uncover an inner main part that defines a kind of chest of drawers comprising a cavity, or main compartment, where several cases TR, substantially shaped as parallelepiped drawers, are inserted, which have internal padded walls adapted to contain equipment and sanitary products.

Each case, after having been inserted into the backpack, can be pulled out from the main cavity independently from the others and remains in its position by virtue of magnetic strips, some of which are attached on a portion of the outer perimeter of each case and the other of which, complementary to the first ones, are fixed to an inner perimeter of the main cavity of the chest of drawers defined by the backpack.

A passive RF TAG with RFID (Radio-Frequency IDentification) technology is associated with each case, having preferably a different color, in which data concerning the type of equipment contained in the respective case (e.g. dressings, medicines, infusion kits, ventilation kits, ropes, anchoring devices, etc.) have been stored beforehand. Such a device, which is provided with a specific logic of management described below, allows loss of a case and the relevant material contained therein to be avoided, and/or to avoid that all the desired cases in connection with the type of mission in which the backpack is involved are not contained in the backpack, and/or that no duplicate of any one of the cases is contained in the backpack.

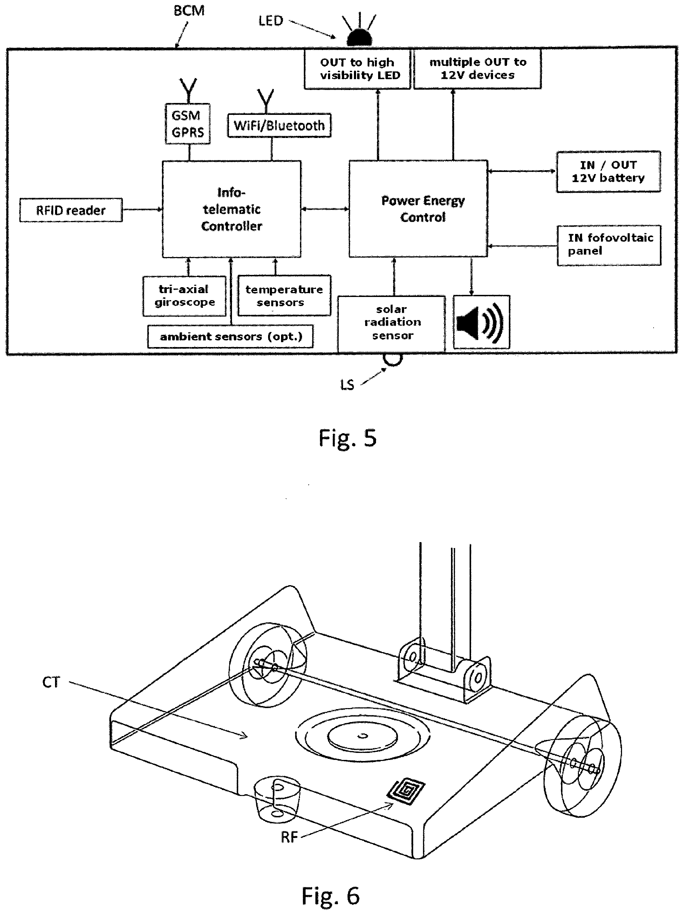

A processing unit called BCM (Backpack Control Module) is received in an upper pocket of the backpack, as shown in a schematic manner in Figure 5, which can be accessed from the outside and/or the inside.

Also a passive (or slave) RFID TAG is associated with the backpack, so that it can be recognized independently from operation and power supply status of the BCM.

The backpack is preferably provided with a detachable transport trolley shown in FIG. 6, which can be detached from and connected to the backpack, and that is also provided with a RFID TAG used for the checklist function described below. This RFID TAG allows the backpack and the trolley to be univocally associated with each other, and tracked for their distance from the RFID reader present in the BCM.

The trolley is equipped with a disk of magnetic material to allow a quick anchorage and the backpack to be aligned with the center of the resting base. Likewise, a small plate of ferromagnetic material is arranged on the base of the backpack, to be attracted by the magnetic disk whenever the backpack is properly positioned on the resting base of the trolley. Therefore, the disk and the small plate couple to each other magnetically.

The processing unit is provided with a RFID interface of the master type, which allows all the RFIDs on board of the backpack as well as the equipment, to be identified one by one, in other words to perform a "checklist".

This query can be made at the request of a user who interfaces with the BCM processing unit by means of a portable device DP (tablet, smartphone or similar device), or it is managed independently by the BCM itself. The presence/type of each case inside the backpack is determined the by the query of the respective RFID TAGs installed on them. Therefore, in the case one or more cases are not present, or a case is wrong or duplicated, a warning message is generated by the BCM together with a corresponding identification code and the description of the error. The anomaly is transmitted by the BCM through its short-range wireless interface to the portable device and/or by a GSM/GPRS (or similar) radio interface to a remote server.

Analogously, if the transport trolley is not detected by the BCM by reading its RFID TAG at the time of the query, the message "carriage not present" is generated and transmitted via the short-range wireless communication and GSM/GPRS channels of the BCM itself. This feature is especially useful when the operator is not located in the area close to the backpack and the trolley. By querying the remote server, he is able to check availability of such equipment in real time.

In the case of "trolley present" and if the backpack is moving, the BCM cyclically checks the distance between its own RFID transceiver (master) and the passive RFID TAG associated with the trolley, for example on the basis of power measurements of the response signal received by the passive TAG. If this distance changes over time, it means that the backpack is moving but it is not anchored to the trolley, so that the trolley is considered to be abandoned/forgotten along the way, by the BCM. In this condition, the BCM activates an acoustic and visual warning in order to warn the operator.

The checklist can be performed manually, that is on request of an operator provided with the said portable device. The information processed by the BCM following to the manual query, are instantly transmitted to the portable device. Conversely, when the query is managed independently by the BCM, the transfer of information to the portable device occurs when a predetermined countdown is expired, preferably lasting 10 to 11 hours. In this manner, the BCM is prevented from transmitting data whenever the backpack is moved. Moreover, just the result of the last query of the RFIDs is sent.

The BCM also carries out a checklist of the on board equipment.

A feature is defined as "semi-automatic" if the BCM performs at least one of the following approaches:
- if a timeout has expired, e.g. when 9 hours (programmable value) have passed since the last checklist, the BCM automatically checks the presence of the cases and determines their distance from the BCM to estimate whether they are within the backpack or nearby. A similar check is carried out for the transport trolley if it needs to be present (as a function of the rescue mission). The BCM also activates an acoustic and visual signal (LED) and, at the same time, transmits the message "execute checklist" to the portable device connected to it, along with the list of the cases (number and type) that are in the backpack at that time. The operator is therefore requested to carry out the manual checklist by verifying visually all the remaining equipment of the backpack that are not provided with a their own RFID TAG;
- if the BCM detects the movement of the backpack through the signals from the tri-axial accelerometer and the gyroscope, and if, at the same time, more than 4 hours (programmable value) are elapsed from the last checklist and the last detected motion, the BCM performs autonomously a check, similarly to the approach described in the previous item, of the presence of the cases and carries out an estimate of their distance from the BCM itself. Preferably, a similar check is carried out for the transport trolley that is also equipped with its own RFID TAG. Also, the BCM activates an acoustic signal, and transmits simultaneously the question message "Do you think it's necessary to complete the check list?" to the portable device connected to it, together with the list of the cases (number and type) that are present in the backpack at that time, but not their content that can only be verified manually if the operator so wishes. This second approach is referred to the typical scenario where the backpack has been used in recent rescue missions and it is deemed to be convenient to re-evaluate, for a greater reliability, if recovery of the previously utilized equipment (single-use and others) occurred. Where the operator does not consider it is necessary to perform the manual, or visual, checklist for the remaining equipment not provided with a RFID TAG, after receiving said message, it is sufficient to bring its extensible RFID TAG (operator's identification number) closer than 10 centimeters from the respective BCM reader within 5 minutes (programmable value) from issuing the acoustic warning (time interval in which the BCM activates reading of the operator RFID TAG). Where, within 5 minutes from activation of the warning, the BCM does not read the operator RFID TAG at less than 10 centimeters from the respective BCM reader, it activates for a second and last time the acoustic warning, and simultaneously transmits again the question message "Do you think it's necessary to carry out a complete checklist?" to the portable device connected to it. If, instead, it is necessary to carry out the checklist, it is sufficient to proceed, even in this case, with the manual check through the visual check of the equipment and the devices contained in the backpack, which have not been tracked by the RFID TAG.

The processing unit comprises a preferably tri-axial accelerometer for detecting a condition of use of the backpack following to the accelerations measured along one or more of the X, Y, Z axes. The backpack motion is detected by processing the reading of the signals coming from said tri-axial accelerometer.

One of the "semi-automatic" checklist approaches described above is just based on the detection of the motion of the backpack. Obviously, if this feature is not implemented, then the BCM may not comprise the accelerometer.

As described above, the BCM processing unit transmits the result of reading the RFID TAGs detected to the said portable device (tablet, smartphone, or equivalent device), and this is done by means of a short-range wireless interface, for example the same RFID, Bluetooth, Wi-Fi or equivalent transceiver (master). According to a further preferred modification of the invention which can be combined with the previous one, the processing unit is also provided with a GSM/GPRS (or similar) radio interface and it can cyclically exchange data with a remote server by sending a set of information that includes also the status of the checklist, including the above-mentioned check of the presence of the cases and the transport trolley.

Regardless of the presence of a GSM/GPRS connection, the data are also stored in the internal storage unit of the processor unit, and possibly transmitted to the remote server by periodic attempts, until the transmission procedure ends successfully.

A portable device, such as a tablet or a smartphone or similar, implements a pre-defined App (from the Anglo-Saxon abbreviation for Application) called "Checklist". The App used for the manual check of the material (if it is not provided with a RFID TAG) contained within the backpack by the operator, and for the automatic check of the equipment provided with a RFID TAG, displays a pre-configured table containing the following data referred to the backpack itself and its contents:
- name or identification number of the device/medicine/medical product;
- amount of the current device/medicine/ medical product;
- expiry date of the device/medicine/medical product;
- notes fields where a free comment can be entered, activated automatically during compilation in the case there are any anomalies (amount below than expected, missing or damaged product).

The App requires the operator entrusted with the checklist to select each item described in the previous items, by indicating any deviation from the requirement set for the health or technical protocol for the type of material: present/absent, amounts and expiry.

The App includes a synthesis page called "Control page" in which the following information are shown, that are also processed in the table previously described and through the data stored in the BCM (of factory and implemented by the supervisor/owner authority of the backpack):
- date, time and operator identification number (ID) referred to the last checklist made and completed;
- backpack configuration (the data is stored in the BCM): as a function of the mission for which it is to be used: primary or secondary medical assistance or technical assistance (e.g. alpine-riverine-speleological). Should the mission not foresee a priori the presence of the transport trolley on the checklist, the line of the table corresponding to the trolley is automatically inhibited. As a consequence, the BCM does not perform under any condition reading of the RFID TAG of the trolley. Also, the number and type of the cases to be included in the backpack are predefined depending on the type of mission;

- any possible difference and deviation of the currently identified material from that of the previous checklist (verified manually and visually by the operator) in order to detect the lack of recovering material/equipment in the missions from the last check;
- name, last name, business identification number (ID), and role of the operator entrusted of the checklist. These information are automatically detected by reading a programmable RFID TAG supplied with the operator and applied by an extensible support (similar to that of the badge holder) to be applied to its professional uniform, in one of its (inner or outer) pockets or in the buckle of a belt. The TAG is read directly by the previously defined portable device, and simultaneously by the RFID reader arranged in the BCM processing unit on board of the backpack;
- identification code (ID) of the backpack, date of its production and possible certifications obtained;
- first time use date, automatically requested by the App at the first access;
- name of the owner authority and/or the responsible person;
- date of last maintenance/technical check (where applicable). An anomaly warning is triggered on the portable device if the check has not been carried out by the scheduled expiry date;
- indication of devices/medicines/medical products expired or close to the expiry date;
- number of checklists completed 100% with a positive result, with respect to the total of the checklists made;
- automatic storage of the GPS coordinates of the backpack, calculated for each checklist.

When a checklist is completed, the portable device automatically sends and stores in the BCM flash memory the data collected through the communication systems. In this manner, it is possible to query manually the BCM from any associated portable device in order to know in real time the exact status of the checklist, that is, the actual content of the backpack at that very moment.

According to a further preferred modification of the invention, which can be combined with those previously described, the backpack comprises one or more pockets made of PCM (Phase Changing Material) insulating material. Each pocket contains a gap within which an eutectic substance of natural origin is placed, having a positive eutectic point (about 35°C), which function is that to heat one or more bags of a solution (e.g. physiological) for injection. Heating of the eutectic substance is obtained by a preferably ultra-flat and made of a metallic alloy electrical resistance, which is powered by the processing unit itself. A temperature sensor is arranged inside said pocket made of insulating material and, preferably, an additional temperature sensor is arranged outside the backpack in order to apply in advance, by acting on the electrical resistance control, sudden changes in the temperature of the external environment, which inevitably affect even the temperature inside the bag with some delay, as a function of the thermal constants of the insulating materials. Both the sensors are connected to the BCM processing unit, which can thus control linearly the power of the electrical resistance and, consequently, the internal heating of the pocket.

Preferably, the processing unit is configured to activate the resistance if, and only if, both the following conditions are verified:
- the last checklist carried out shows the presence of at least one bag of solution for injection,
- the inside temperature of the pocket is 4°C below the rated temperature of 35°C (parameter that can be set on the BCM) and at the same time the motion of the backpack is automatically detected during the last hour (this is also a configurable parameter on the BCM) starting from the data measured by the tri-axial accelerometer and the gyroscope, both integrated into the BCM processing unit as described above.

Thus, according to preferred modifications of the invention where at least one temperature sensor inside the backpack and one environment sensor are provided, the processing unit implements at least one of the following features:
- detecting the inner temperature of the backpack in order to preserve any content of the backpack which is sensitive to high and/or low temperatures, for example medicines and thermolable products, or to sudden temperature gradients (dT/dt, where T is the temperature and t is the time). If a preset threshold, which can be changed via SW, is exceeded, an acoustic and/or visual signal is transmitted to the portable device, and/or the BCM emits a predefined warning sound;
- detecting the environment temperature in order to safeguard the health of the staff located in the immediate vicinity of the backpack, by signaling high and/or low temperatures that may have a harmful effect on the human body, as well as the sudden temperature gradients (dT/dt, where T is the temperature and t is the time) due to unforeseeable external phenomena (e.g. chemical reactions or combustion of materials). In the case a preset threshold, which can be changed via SW, is exceeded, an acoustic and/or visual signal is transmitted to the portable device, or the BCM emits a preconfigured warning sound.
Preferably, the portion of the backpack that allows the main cavity to be accessed, includes a cover CV that comprises two or more rigid rectangular bands hinged on one another, on which photovoltaic modules PV are applied, which are connected with said processing unit for local generation of electric power.

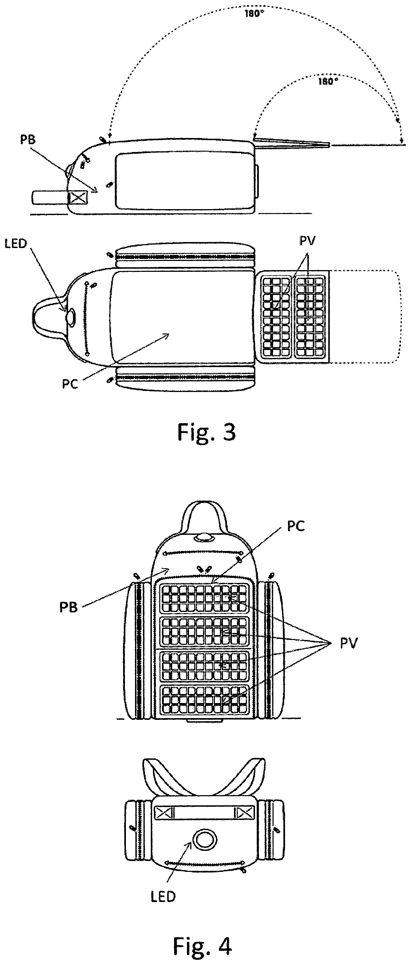

Figure 3 shows the movement of the cover from an almost fully closed position of the main compartment PC, shown by a continuous line, and a fully open position in which the cover is represented by a dashed line. In this last configuration, the cover is packed with its two or more rigid bands overlapping each other, in order to allow access to the main compartment. Keeping the cover in the packed position is ensured by a magnetic plate opposed to a corresponding ferromagnetic support, which is placed in the inner lining of the cover itself.

According to a further preferred modification of the invention which can be combined with those previously described, a high brightness and efficiency LED signaling light (single LED or LED group), simply indicated as "LED", is arranged on the top of the backpack to allow the position of the backpack to be visually indicated under low visibility conditions, as well as a brightness sensor LS, as shown in Figures 4 and 5, both of which are connected to the BCM processing unit to carry out at least one of the features described below.

Also, a multiple socket is provided in a recess of the backpack for supplying power to possible external user devices to be powered by the batteries/photovoltaic module of the backpack.

Figure 5 schematically shows a preferred modification of the processing unit, called BCM, associated with the backpack.

The processing unit can be schematized by means of two main macro-blocks:
- Power Energy Control, e
- Info-Telematic Controller.

The first macro-block of the processing unit, called "Power Energy Control", manages the connection between the photovoltaic panels PV and the electrical power supply of the backpack, which is necessary for the operation of the BCM itself, of the various sensors and electrical devices connected to it, including possible external users. Moreover, it controls operation of the LED signaling light and activation of the acoustic warning integrated in the BCM.

The acoustic warning and the LED can be used jointly by the BCM to provide anomaly signals or indicate actions required to the operator.

The main functions performed by the "Power Energy Control" are:
- ensuring power supply priority for the photovoltaic panel when the intensity of solar lighting is sufficient to generate electrical power;
- checking the multiple external power supply socket, having the possibility of programming the power priority of each port of the socket, and therefore between the devices connected to it, depending on the charge status of the backpack battery and/or the power generated by the photovoltaic module;
- ensuring power supply of the devices directly connected to the processing unit with respect to the external devices connected to the multiple socket, depending on the charge status of the battery;
- power supplying the Info-telematic Controller;
- interacting with the Info-telematic Controller, from which the switching-on and -off command for the high brightness and visibility LED system (single LED or LED group) comes, according to the logic described in the next item;
- transmitting information to the Info-telematic Controller about the charge status of the on board battery.

The second macro-block, the "Info-telematic Controller", integrates or is connected to:
- long-range wireless GSM/GPRS/3G/LTE etc. communication systems,
- short-range Wi-Fi, Bluetooth wireless communication systems, and RFID reader,
- a GPS tracking receiver,
- temperature sensors and possible humidity sensors,
- a solar radiation sensor,
- a preferably tri-axial accelerometer, and a gyroscope to determine operating condition of the backpack,
- processing and control electronics to interface with the above listed components and with the Power Energy Control, as well as to process, transmit and receive data to and from a portable device and a remote server,
- a solid state or flash-type internal memory for storing the data acquired and loaded through the portable device.

The processing unit controls, through the Power Energy Control, switching-on and -off the LED system according to at least one of the following modes:
- **"Manual" mode:** by a "Led" App installed on the portable device, the LED light is manually activated. The Info-telematic Controller, after receiving the switching-on/off command through a short-range interface of the portable device, forwards this information to the Power Energy Control via an internal communication bus inside the processing unit that connects the two macro-blocks. The switching-on command issued by the portable device, can be ignored by the Power Energy Control when the battery charge status is below a programmable pre-defined threshold (e.g. 25%). In addition, the LED system switching-off of can be forced independently by the Power Energy Control when the battery charge status is below such a predetermined threshold (e.g. 20%) and/or when a countdown is expired, the duration of which (for example 30 min.) can be programmed;
- **"Auto" mode:** the AUTO mode can be selected by using the portable device interface, always through the above App. This mode is preferably active by default. The LED system switching-on takes place if two conditions occur at the same time:
- the distance between the RFID TAG associated with the BCM and used as reference (e.g. a fixed point on a helicopter) and the backpack is greater than a predetermined distance, for example 5 meters; and
- the radiation sensor detects an ambient light condition below a predetermined threshold. As above, the LED system switching-off can be forced by the Power Energy Control depending on the charge status of the battery and/or when a programmable predefined time (e.g. 30 mins.) has elapsed.

The distance between the backpack and the TAG can be measured in a number of ways, for example by measuring the power signal in the wireless data connection between the one and the other, or by estimating the respective positions obtained from the processing unit on the basis of its own GPS receiver, and from the portable device on the basis of the corresponding GPS receiver, respectively. According to a further preferred modification of the invention, which can be combined with those described above, if the measured distance between the RFID reader of the BCM and the corresponding RFID TAG associated with it, which is used as a fixed reference (for example placed in the health compartment of the helicopter) is less than 3 meters (programmable value), the BCM automatically deactivates the short and long-range communication channels to avoid any risk of electromagnetic interference (mainly radiation susceptibility) with the electrical and electronic devices on board the helicopter. Likewise, the above channels are reactivated automatically when both (AND) the following conditions are verified:
- a backpack motion is detected followed by the immediate reading of all the RFID TAGs associated with the BCM, and
- the distance between the RFID reader of the BCM and the corresponding RFID TAG used as a fixed reference (e.g. placed in the helicopter health compartment) is greater than 3 meters (programmable value). This condition identifies the removal of the backpack from the helicopter, so that there are no risks, even if remote, of an electromagnetic interference. A similar function is performed in the presence of an ARVA device (Appareil de Recherche de Victimes en Avalanche) or similar, in the case of radiofrequency signals. In this case, the ARVA device will be equipped with an RFID TAG and if its distance from the BCM is less than 100 meters (configurable parameter), the above-mentioned wireless communication channels are deactivated. Reactivation of the latter occurs when the distance between the RFID TAG and the BCM becomes again more than 150 meters (also this value can be changed).

According to a preferred modification of the invention, for both the Manual or Auto modes, if the tri-axial accelerometer detects that the backpack is kept stationary for a time longer than a predetermined threshold (e.g. 5 min.), the ON/OFF flash frequency changes so as to reduce the LED power consumption.

According to preferred modifications of the invention, the processing unit implements at least one of the following features, based on the gyroscope and the tri-axial accelerometer:
- sending a message to a remote server and, at the same time, activating the acoustic warning from the BCM, to inform about an impact of the backpack against a fixed or mobile obstacle, i.e. that a deceleration threshold, which can be adjusted via the SW, has been exceeded for at least one of the three axes (X, Y and Z);
- sending a message to a remote server to indicate the backpack is in motion, as a result of detecting its position by the relevant GPS receiver or via the tri-axial accelerometer;
- activating an acoustic and/or visual message in the case a limit swing and/or rotation of the backpack, and presumably also of the operator wearing the backpack during a winch operation (e.g. by an helicopter or in a cave), is exceeded. The aim is to ensure safety of the operator who is suspended, in connection with possible impacts against surrounding obstacles or with the irreversible damage of the cable. Moreover, excessive oscillations make complex reaching of a predetermined area on the surface or subsoil.

According to a preferred modification of the invention, the BCM and the portable device are configured to implement a feature called **"follow me".** In particular, the processing unit (BCM) of the backpack is queried by a special App installed on the portable device, and the distance between the backpack and the RFID TAG worn by the operator is calculated, with a programmable frequency. The distance can be calculated on the basis of the signal strength between the processing unit BCM of the backpack and the RFID TAG. When this distance is more than 30 meters (programmable value), an acoustic and/or visual warning is triggered by the BCM indicating a possible involuntary abandonment of the backpack. At the same time, the BCM sends the same warning to the associated portable device, through a short-range communication channel of it.

The processing unit of the backpack may preferably implement a feature called **"rescue me".** The purpose of such a function is to identify a situation where the backpack can be buried (e.g. under a snowy mantle, a mound of earth or rubbles) as a result of a sudden event during a rescue mission. The BCM acquires real-time information about the time (through the internal GPS) and the ambient light using the solar radiation sensor it is equipped with. When the latter detects a sudden absence of light in a daylight time and simultaneously a sudden motion of the backpack, detected by the gyroscope and the tri-axial accelerometer, followed by a total absence of motion or a minimum variations thereof (which can be associated with the burial of the backpack and the operator) for a preset and programmable time (e.g. 5 min.), the BCM transmits a warning signal to the portable device and/or to the remote server, indicating the possibility that the backpack is buried. At the same time, the BCM itself sends the last position of the backpack detected through the GPS, when the above conditions occur.

Preferably, a specific App installed on the portable device, capable of receiving said message and of emitting, through the intrinsic features of the portable device, an acoustic signal and a visual signal showing the last position of the backpack at the time of sending the warning message.

Advantageously, a rescue team is able to reach the position tracked by the GPS and, by virtue of a Recco® plate, known per se, sewn on the outer surface of the backpack, it is able to precisely determine the position in order to rescue it. After the warning signal, the BCM acquires every 5 min. (programmable value) the temperature data detected by the external temperature sensor mounted on board the backpack connected to it. Again, such a time interval is a programmable parameter.

Preferably, the BCM processes the temperature data by calculating thermal variations from the last measurement obtained just before the total absence of light. In the unfortunate case where the backpack is buried by a snow avalanche, over time the temperature reaches an asymptotic value corresponding to the temperature of the snowy layer surrounding the backpack. This information about the change of the temperature in the time is used by the BCM to improve the reliability of the information about the fact that the backpack is actually buried. Depending on the temperature trend as a function of the time, the "buried backpack" condition is transmitted by the BCM to the portable device and/or the remote server. The elements and features shown in the various preferred embodiments may be combined with each other without, however, departing from the scope of protection of the present invention, as defined by the appended claims.

## Claims

1. Professional backpack, particularly for technical and/or specialized medical rescue missions, wherein it comprises:
- a cavity, or main compartment,
- one or more cases (TR) adapted to be inserted in or extracted from said main cavity one independently from each other, a passive RFID TAG being associated with each case,
- a processing unit (BCM) associated with the backpack, comprising a master RFID interface to query said passive RFID TAG and configured to perform a "checklist" of the cases (TR) actually on board the backpack,
**characterized in that** said backpack further comprises a front opening that can be closed preferably by means of one or two zippers symmetrical to each other with respect to the longitudinal axis of the backpack, and **in that** said processing unit is further provided with an accelerometer sensor, and it is configured to perform said checklist when said accelerometer sensor reveals that the backpack is moving.

2. Backpack according to claim 1, **characterized in that** it further comprises a trolley (CR) for transporting the backpack, which can be detached from and connected to the backpack, in which the trolley is provided with a support base (CT) for the backpack, having a first shaped portion of magnetic material, and **in that** the said backpack comprises a second portion of magnetic or ferromagnetic material so shaped to be complementary to said first portion so as to allow the backpack to be quickly and affordably anchored on said support base, and in which said trolley is also provided with a passive RFID TAG (RF), and wherein said processing unit is configured to detect also the presence of said trolley by query said respective RFID TAG.

3. Backpack according to claim 1 or 2, **characterized in that** said processing unit further comprises short-range data transmission means (RFID, Bluetooth, Wi-Fi or equivalent) configured to transmit a result of said checklist to a portable processing device (DP) such as a smartphone or a tablet.

4. Backpack according to any one of claims 1 to 3, **characterized in that** said processing unit (BCM) is configured so as to transmit said checklist to said portable processing device after a timeout expires.

5. Backpack according to any one of claims 1 to 4, **characterized in that** it further comprises a pocket coated by a PCM insulating material containing an eutectic substance and comprising an electrical resistance and a temperature sensor, both the resistance and the temperature sensor being connected to said processing unit (BCM), and in which said processing unit is configured so as to control said electrical resistance in order to keep said substance at a predetermined settable temperature.

6. Backpack according to any one of claims 1 to 6, **characterized in that** said processing unit performs said control of said electrical resistance when it detects simultaneously a motion of said backpack by means of said accelerometer sensor.

7. Backpack according to any one of claims 1 to 6, **characterized in that** said processing unit (BCM) incorporates:
- a gyroscope and a tri-axial accelerometer,
- acoustic and/or visual (LED) signaling means installed on the outside of the backpack,
- long range means of communication of the GSM, 3G, or similar type, in which the processing unit is configured to send a warning message to a remote server by said long-range means of communication, and to activate said acoustic and/or visual signaling means when said gyroscope and tri-axial accelerometer indicate an impact of the backpack against an obstacle.

8. Backpack according to claim 7, **characterized in that** said processing unit further comprises a GPS receiver, and in which it is configured to integrate in said warning message also a position of the backpack obtained by means of said GPS receiver.

9. Backpack according to any one of claims 1 to 8, wherein said processing means incorporate:
- an ambient lighting sensor installed on the outside of the backpack, and
- a tri-axial accelerometer sensor,
and in which said processing means are configured so as to transmit a warning signal to a portable device and/or to a remote server, to indicate the burial of the backpack when, after a sudden motion of the backpack is detected, a sudden reduction of the light radiation incident on said ambient lighting sensor is identified.

10. Safety equipment comprising a backpack according to any one of claims 8 to 9, **characterized in that** it comprises a portable processing unit such as a tablet or a smartphone, provided with an App interacting with the portable processing unit of said backpack, said App being configured to perform at least one of the following functions:
- automatic or manual activation/deactivation of said signaling means (LED), the transmission of the switching-on/off command for said signaling means (LED) occurring by a short-range interface through the App installed on the portable device, in which the switching-on command can be ignored by the processing unit when the battery charge status is below a predetermined programmable threshold, and in which switching-off of said signaling means (LED) can be forced autonomously by the processing unit when the charge status of the relevant battery is below such a predetermined programmable threshold, and/or when an adjustable countdown expires, and/or in which it is possible to select an AUTO mode, through said App, from the interface of the portable device, in which case switching-on said signaling means (LED) takes place if the following two conditions occur simultaneously:
- the distance between the TAG RFID used as a reference (e.g. a fixed point on a helicopter) associated with the processing unit and the backpack is greater than a predetermined distance; and
- the radiation sensor detects an ambient lighting condition below a predetermined threshold;
- querying the backpack processing unit (BCM) by means of the portable device, in order to calculate cyclically, according to a programmable parameter, the distance between the backpack and a RFID TAG worn by the operator ("follow me") ;
- receiving a checklist from said processing unit, and displaying on the display of the portable processing unit the checklist including at least one of the following data:
- date, time and identification number (ID) of the operator, in connection with the last checklist made and completed;
- backpack configuration as a function of the mission for which it is set up;
- possible differences and deviations between the identified material (manually and visually checked by the operator) with respect to that of a previous checklist, in order to detect a failure to recover the material/equipment of the missions as from the last check;
- name, last name, business identification number (ID), and role of the operator responsible for the checklist;
- backpack identification code (ID), date of production thereof and any certifications obtained;
- first-time use date automatically requested by the App at the first access;
- name of the owner authority and/or the responsible person;
- date of the last maintenance/technical check (where applicable); where no check has been made by the scheduled expiry date, an error message is triggered on the portable device;
- indication of devices/medicines/health care products expired or close to the expiry date;
- number of checklists completed 100% with a positive result compared to the total of checklists made;
- automatically storing the backpack GPS coordinates calculated during each checklist;
- manual activation of said signaling means (LED);
- receiving a message from the backpack processing means, containing a backpack position calculated by the processing unit of the same backpack, and calculating the position thereof by using its own GPS receiver, and displaying on a display of the portable device a position of the backpack at the time said warning message is sent by the backpack.

## Patentansprüche

1. Professioneller Rucksack, insbesondere für technische und/oder spezialisierte medizinische Hilfseinsätze, umfassend:
- einen Innenraum oder ein Hauptabteil,
- einen oder mehrere Koffereinsätze (TR), die geeignet sind, unabhängig voneinander in den Hauptraum eingesetzt oder aus diesem herausgenommen zu werden, wobei jedem Koffer ein passives RFID TAG (Hochfrequenz-Identifikations-Kennzeichen) zugeordnet ist,
- eine Auswerteeinheit (BCM), die mit dem Rucksack verbunden ist und eine RFID-Masterschnittstelle zum Abfragen des passiven RFID TAG hat und so ausgebildet ist, dass sie eine Checkliste der aktuell an Bord des Rucksacks befindlichen Koffereinsätze (TR) erstellen kann,
**dadurch gekennzeichnet, dass** der Rucksack ferner eine vordere Öffnung hat, die vorzugsweise durch einen Reißverschluss oder zwei zur Längsachse des Rucksacks symmetrische Reißverschlüsse verschließbar ist, und dass die Auswerteinheit ferner einen Beschleunigungssensor hat und so ausgebildet ist, dass sie die Checkliste erstellt, wenn der Beschleunigungssensor eine Bewegung des Rucksacks feststellt.

2. Rucksack nach Anspruch 1, **dadurch gekennzeichnet, dass** dieser ferner ein Fahrgestell (CR) für den Transport des Rucksacks hat, das an diesem angebracht oder von diesem abgenommen werden kann und eine Tragbasis (CT) für den Rucksack hat, welche einen ersten Formatabschnitt aus magnetischem Material hat, wobei der Rucksack einen zweiten Abschnitt aus magnetischem oder ferromagnetischem Material hat, der eine mit dem ersten Abschnitt komplementäre Form zum raschen und sicheren Fixieren auf der Tragbasis hat, welche ebenfalls mit einem passiven RFID TAG versehen ist, wobei die Auswerteeinheit so konfiguriert ist, dass sie bei Abfrage des RFID TAG auch die Anwesenheit des Fahrgestells feststellt.

3. Rucksack nach Anspruch 1 oder 2, **dadurch gekennzeichnet, dass** die Auswerteeinheit ferner Datenübertragungsmittel mit kurzer Reichweite (RFID, Bluetooth, Wi-Fi, W-LAN) oder dergleichen hat, die so konfiguriert sind, dass sie ein Ergebnis der Checkliste an eine tragbare Auswertevorrichtung (DP) wie z. B. ein Smartphone oder ein Tablet übertragen.

4. Rucksack nach einem der vorhergehenden Ansprüche, **dadurch gekennzeichnet, dass** die Auswerteeinheit (BCM) so konfiguriert ist, dass sie die Checkliste nach Ablauf einer Zeitsperre an die tragbare Auswertevorrichtung überträgt.

5. Rucksack nach einem der vorhergehenden Ansprüche, **dadurch gekennzeichnet, dass** dieser ferner eine Tasche aufweist, die mit einem PCM isolierenden Material beschichtet ist, das eine eutektische Substanz enthält und einen elektrischen Widerstand sowie einen Temperatursensor aufweist, welche beide mit der Auswerteeinheit (BCM) verbunden sind, welche so konfiguriert ist, dass sie den elektrischen Widerstand so steuert, dass die Substanz auf einer vorbestimmten, einstellbaren Temperatur gehalten wird.

6. Rucksack nach einem der vorhergehenden Ansprüche, **dadurch gekennzeichnet, dass** die Auswerteeinheit die Steuerung des elektrischen Widerstands durchführt, wenn sie gleichzeitig über den Beschleunigungssensor eine Bewegung des Rucksacks feststellt.

7. Rucksack nach einem der vorhergehenden Ansprüche, **dadurch gekennzeichnet, dass** die Auswerteeinheit (BCM) aufweist:
- ein Gyroskop mit dreiachsigem Beschleunigungsmesser,
- akustische und/oder visuelle (LED) Anzeigemittel, die an der Außenseite des Rucksacks installiert sind,
- Übertragungsmittel großer Reichweite vom Typ GSM, 3G o. dgl., wobei die Auswerteeinheit so konfiguriert ist, dass sie durch die Übertragungsmittel großer Reichweite an einen Fernserver ein Warnsignal aussendet und die akustischen und/oder visuellen Anzeigemittel aktiviert, wenn das Gyroskop und der dreiachsige Beschleunigungsmesser einen Stoß des Rucksacks gegen ein Hindernis anzeigen.

8. Rucksack nach Anspruch 7, **dadurch gekennzeichnet, dass** die Auswerteeinheit ferner einen GPS-Receiver hat und so konfiguriert ist, dass in dem Warnsignal auch eine Positionsangabe des Rucksacks integriert ist, die von dem GPS-Receiver ermittelt wurde.

9. Rucksack nach einem der vorhergehenden Ansprüche, **dadurch gekennzeichnet, dass** die Auswerteeinheit umfasst:
- einen Umgebungslichtsensor, der an der Außenseite des Rucksacks angebracht ist,
- einen dreiachsigen Beschleunigungssensor, wobei die Auswerteeinheit so konfiguriert ist, dass sie ein Warnsignal an eine tragbare Vorrichtung und/oder an einen Fernserver aussendet, das einen verschütteten Ort des Rucksacks angibt, wenn nach Feststellung einer plötzlichen Bewegung des Rucksacks ein plötzlicher Abfall der Lichtstrahlung auf den Umgebungslichtsensor festgestellt wird.

10. Sicherheitsausrüstung mit einem Rucksack nach Anspruch 8 oder 9, **dadurch gekennzeichnet, dass** diese eine tragbare Auswerteeinheit wie z. B. ein Tablet oder ein Smartphone aufweist, die mit einer App für die Interaktion mit der tragbaren Auswerteeinheit des Rucksacks versehen und so konfiguriert ist, dass sie wenigstens eine der nachstehenden Funktionen durchführt:
- automatische oder manuelle Aktivierung/Deaktivierung der Anzeigemittel (LED), Übertragung des Einschalt-/Ausschaltbefehls für die Anzeigemittel (LED) durch die App, die in der tragbaren Vorrichtung über ein Interface mit kurzer Reichweite installiert ist, wobei der Einschaltbefehl von der Auswerteeinheit ignoriert werden kann, wenn der Ladezustand der Batterie unter einem vorbestimmten, programmierbaren Schwellenwert liegt, und wobei der Ausschaltbefehl für die Anzeigemittel (LED) durch die Auswerteeinheit autonom forciert werden kann, wenn der Ladezustand der betroffenen Batterie unter dem vorbestimmten, programmierbaren Schwellenwert liegt, und/oder wenn ein einstellbarer Countdown abgelaufen ist und/oder wenn über die App ein AUTO-Modus von dem Interface der tragbaren Vorrichtung ausgewählt werden kann, in welchem Fall die Anzeigemittel (LED) eingeschaltet werden, sofern die beiden nachstehenden Bedingungen gleichzeitig erfüllt sind:
- der Abstand zwischen dem als Referenz dienenden (z. B. ein Festpunkt auf einem Helikopter-) TAG RFID, der mit der Auswerteeinheit und dem Rucksack verbunden
ist, ist größer als ein vorbestimmter Abstand und
- der Strahlungssensor ermittelt einen Umgebungslichtzustand unterhalb eines vorbestimmten Schwellenwertes;
- Abfrage der Rucksack-Auswerteeinheit (BCM) mittels der tragbaren Vorrichtung, um nach einem programmierbaren Parameter zyklisch den Abstand zwischen dem Rucksack und einem von der Bedienungsperson getragenen RFID TAG ("follow me") zu berechnen;
- Empfang einer Checkliste von der Auswerteeinheit und Wiedergabe der Checkliste auf dem Display der tragbaren Auswerteeinheit mit wenigstens einem der folgenden Daten:
- Datum, Zeit und Identifikationsnummer (ID) der Bedienungsperson in Verbindung mit der zuletzt erstellten und vervollständigten Checkliste,
- Konfiguration des Rucksacks in Funktion des Einsatzes, für den er bestimmt ist, - mögliche Unterschiede und Abweichungen zwischen dem registrierten Material (von der Bedienungsperson manuell und visuell überprüft) und der vorangegangenen Checkliste, um eine fehlerhafte Verwendung von Material/Ausrüstung der Einsätze gemäß vorangegangenem Check zu ermitteln,
- Name, Vorname, Geschäftsnummer (ID) und Funktion der für die Checkliste verantwortlichen Person,
- Rucksack-Identifikationsnummer (ID) mit Herstellungsdatum und erhaltenen Zertifikaten,
- Datum des ersten Einsatzes, das von der App beim ersten Einsatz automatisch abgefragt wird,
- Name der Besitzerorganisation und/oder der verantwortlichen Person,
- Datum des letzten Wartungs- und Funktionschecks (falls vorgesehen); sofern keine Überprüfung innerhalb des vorgeschriebenen Zeitraumes durchgeführt wurde, wird eine Fehlermeldung auf der tragbaren Vorrichtung eingetragen,
- Angabe von Vorrichtungen/Arzneimitteln/Gesundheitsprodukten, die am oder nahe am Ablaufdatum abgelaufen sind;
- Nummer der Checklisten, die zu 100 % ein positives Ergebnis im Vergleich zu allen erstellten Checklisten erbrachten,
- automatische Speicherung der GPS-Koordinaten, die bei jeder Checkliste ermittelt wurden;
- manuelle Aktivierung der Anzeigemittel (LED);
- Empfang einer Mitteilung von den Auswertemitteln, die die von der Auswerteeinheit errechnete Position für den aktuellen Rucksack enthält, sowie Berechnung der Position durch Einsatz des eigenen GPS-Receivers, Anzeige der Position auf dem Display der tragbaren Vorrichtung im Zeitpunkt der Abgabe einer Warnmitteilung durch den Rucksack.

## Revendications

1. Sac à dos professionnel, notamment pour des missions de sauvetage technique et/ou du domaine médical spécialisé, qui comprend :
- une cavité, ou compartiment principal,
- une ou plusieurs coffrets (TR) adaptés pour être insérés dans ladite cavité principale ou extraits de celle-ci, indépendamment les uns des autres, une étiquette RFID passive étant associée à chaque coffret,
- une unité de traitement (BCM) associée au sac à dos, comprenant une interface RFID maîtresse pour interroger ladite étiquette RFID passive et configurée pour effectuer une "check-list" des coffrets (TR) effectivement présents dans le sac à dos, **caractérisé en ce que** ledit sac à dos comprend en outre une ouverture avant qui peut être fermée de préférence au moyen d'une ou de deux fermetures à glissières symétriques l'une par rapport à l'autre par rapport à l'axe longitudinal du sac à dos, et **en ce que** ladite unité de traitement est en outre munie d'un capteur accéléromètre, et **en ce qu'**elle est configurée pour effectuer ladite check-list lorsque ledit capteur accéléromètre révèle que le sac à dos est mobile.

2. Sac à dos selon la revendication 1, **caractérisé en ce qu'**il comprend en outre un chariot (CR) pour le transport du sac à dos, qui peut être détaché du sac à dos et relié à celui-ci, le chariot étant muni d'une embase de support (CT) pour le sac à dos, ayant une première partie conformée faite en matériau magnétique, et **en ce que** ledit sac à dos comprend une deuxième partie en matériau magnétique ou ferromagnétique conformé de façon à être complémentaire à ladite première partie afin de permettre au sac à dos d'être ancré rapidement et économiquement à ladite embase de support, et ledit chariot étant également muni d'un étiquette RFID passive (RF), et ladite unité de traitement étant configurée pour détecter également la présence dudit chariot par interrogation de ladite étiquette RFID respective.

3. Sac à dos selon la revendication 1 ou la revendication 2, **caractérisé en ce que** ladite unité de traitement comprend en outre des moyens de transmission de données à courte portée (RFID, Bluetooth, Wi-Fi ou équivalent) configurés pour transmettre un résultat de ladite check-list à un dispositif de traitement portable (DP) tel qu'un smartphone ou une tablette.

4. Sac à dos selon l'une quelconque des revendications 1 à 3, **caractérisé en ce que** ladite unité de traitement (BCM) est configurée de manière à transmettre ladite check-list audit dispositif de traitement portable après expiration d'un délai.

5. Sac à dos selon l'une quelconque des revendications 1 à 4, **caractérisé en ce qu'**il comprend en outre une poche revêtue d'un matériau isolant PCM contenant une substance eutectique et comprenant une résistance électrique et un capteur de température, le capteur de résistance et le capteur de température étant tous deux connectés à ladite unité de traitement (BCM), et dans lequel ladite unité de traitement est configurée pour commander ladite résistance électrique afin de maintenir ladite substance à une température réglable prédéterminée.

6. Sac à dos selon l'une quelconque des revendications 1 à 6, **caractérisé en ce que** ladite unité de traitement effectue ladite commande de ladite résistance électrique lorsqu'elle détecte simultanément un mouvement dudit sac à dos au moyen dudit capteur accéléromètre.

7. Sac à dos selon l'une quelconque des revendications 1 à 6, **caractérisé en ce que** ladite unité de traitement (BCM) inclut :
- un gyroscope et un accéléromètre triaxial,
- des moyens de signalisation acoustique et/ou visuelle (LED) installés à l'extérieur du sac à dos,
- des moyens de communication à longue portée du type GSM, 3G, ou similaire, l'unité de traitement étant configurée pour envoyer un message d'avertissement à un serveur distant par lesdits moyens de communication à longue portée, et pour activer lesdits moyens de signalisation acoustique et/ou visuelle lorsque ledit gyroscope et ledit accéléromètre triaxial indiquent un impact du sac à dos contre un obstacle.

8. Sac à dos selon la revendication 7, **caractérisé en ce que** ladite unité de traitement comprend en outre un récepteur GPS, et dans lequel elle est configurée pour intégrer dans ledit message d'avertissement également une position du sac à dos obtenue au moyen dudit récepteur GPS.

9. Sac à dos selon l'une quelconque des revendications 1 à 8, dans lequel lesdits moyens de traitement incluent :
- un capteur d'éclairage ambiant installé à l'extérieur du sac à dos, et
- un capteur accéléromètre triaxial,
et dans lequel lesdits moyens de traitement sont configurés de manière à transmettre un signal d'avertissement à un dispositif portable et/ou à un serveur distant, pour indiquer l'enfouissement du sac à dos lorsque, après qu'un mouvement soudain du sac à dos ait été détecté, une réduction soudaine du rayonnement lumineux incident sur ledit capteur d'éclairage ambiant soit identifiée.

10. Équipement de sécurité comprenant un sac à dos selon l'une quelconque des revendications 8 à 9, **caractérisé en ce qu'**il comprend une unité de traitement portable telle qu'une tablette ou un smartphone, munie d'une application interagissant avec l'unité de traitement portable dudit sac à dos, ladite application étant configurée pour exécuter au moins une des fonctions suivantes :
- activation/désactivation automatique ou manuelle desdits moyens de signalisation (LED), la transmission de la commande de mise en fonctionnement/hors fonctionnement pour lesdits moyens de signalisation (LED) se produisant par une interface à courte portée à travers l'application installée sur le dispositif portable, la commande de mise en fonctionnement pouvant être ignorée par l'unité de traitement lorsque l'état de charge des batteries est inférieur à un seuil programmable prédéterminé, et la mise hors fonctionnement desdits moyens de signalisation (LED) pouvant être forcée de manière autonome par l'unité de traitement lorsque l'état de charge de la batterie concernée est inférieur à un tel seuil programmable prédéterminé, et/ou lorsqu'un compte à rebours réglable expire, et/ou dans lequel il est possible de sélectionner un mode AUTO, via ladite App, depuis l'interface du dispositif portable, auquel cas la mise en fonctionnement des moyens de signalisation (LED) est effectuée si les deux conditions suivantes se produisent simultanément :
- la distance entre le étiquette RFID utilisée comme référence (par exemple un point fixe sur un hélicoptère) associée à l'unité de traitement et le sac à dos est supérieure à une distance prédéterminée ; et
- le capteur de rayonnement détecte une condition d'éclairage ambiant inférieure à un seuil prédéterminé ;
- interrogation de l'unité de traitement du sac à dos (BCM) au moyen du dispositif portable, afin de calculer cycliquement, selon un paramètre programmable, la distance entre le sac à dos et une étiquette RFID portée par l'opérateur ("follow me") ;
- la réception d'une check-list en provenance de ladite unité de traitement et l'affichage sur l'écran de l'unité de traitement portable de la check-list comprenant au moins une des données suivantes :
- la date, l'heure et le numéro d'identification (ID) de l'opérateur, en relation avec la dernière check-list établie et complétée ;
- la configuration du sac à dos en fonction de la mission pour laquelle il est préparé ;
- les différences et les écarts éventuels entre le matériel identifié (contrôlé manuellement et visuellement par l'opérateur) et celui d'une check-list précédente, afin de détecter l'impossibilité de récupérer le matériel/équipement des missions à compter de la dernière vérification ;
- le nom, prénom, nom de famille, numéro d'identification de l'entreprise et rôle de l'opérateur responsable de la check-list ;
- le code d'identification du sac à dos (ID), sa date de production et toute certification obtenue ;
- la date de première utilisation demandée automatiquement par l'application lors du premier accès ;
- le nom de l'autorité propriétaire et/ou de la personne responsable ;
- la date de la dernière maintenance/du dernier contrôle technique (le cas échéant) ; si aucune vérification n'a été effectuée avant la date d'expiration prévue, un message d'erreur est déclenché sur l'appareil portable ;
- l'indication des instruments / médicaments / produits de soins de santé périmés ou proches de la date de péremption ;
- le nombre de check-lists effectuées à 100% avec un résultat positif par rapport au nombre total de check-lists établies ;
- le stockage automatique des coordonnées GPS du sac à dos calculées lors de chaque check-list ;
- l'activation manuelle desdits moyens de signalisation (LED) ;
- la réception d'un message provenant du moyen de traitement du sac à dos, contenant une position du sac à dos calculée par l'unité de traitement du même sac à dos, et le calcul de sa position en utilisant son propre récepteur GPS, et l'affichage sur un écran du dispositif portable d'une position du sac à dos au moment où ledit message d'avertissement est envoyé par le sac à dos.
